## Europäisches Patentamt
## European Patent Office
## Office européen des brevets

(19)

(11) Veröffentlichungsnummer: **0 121 702**
**B1**

(12) **EUROPÄISCHE PATENTSCHRIFT**

(45) Veröffentlichungstag der Patentschrift:
**17.12.86**

(51) Int. Cl.⁴: **C 07 C 55/07, C 07 C 51/41**

(21) Anmeldenummer: **84101757.7**

(22) Anmeldetag: **20.02.84**

(54) **Verfahren zum Ausfällen von Americiumoxalat aus einer salpetersauren Ausgangslösung von Americiumnitrat.**

(30) Priorität: **03.03.83 DE 3307600**

(43) Veröffentlichungstag der Anmeldung:
**17.10.84 Patentblatt 84/42**

(45) Bekanntmachung des Hinweises auf die Patenterteilung:
**17.12.86 Patentblatt 86/51**

(84) Benannte Vertragsstaaten:
**DE FR GB**

(56) Entgegenhaltungen:
**US-A-2 872 467**
**US-A-3 781 404**

(73) Patentinhaber: **ALKEM GMBH, Postfach 110069, D-6450 Hanau 11 (DE)**

(72) Erfinder: **Baumann, Siegfried, Am Kimmelsteich 15, D-8757 Karlstein (DE)**
Erfinder: **Westermann, Gerd, Platanenring 31, D-6458 Rodenbach 1 (DE)**
Erfinder: **Gärtner, Franz, Friedrich- Ebert- Strasse 104, D-6454 Bruchköbel 1 (DE)**

(74) Vertreter: **Mehl, Ernst, Dipl.- Ing., Postfach 22 01 76, D-8000 München 22 (DE)**

EP 0 121 702 B1

LIBER. STOCKHOLM 1986

## Beschreibung

Die Erfindung betrifft ein Verfahren zum Ausfällen von Americiumoxalat aus einer salpetersauren Ausgangslösung von Americiumnitrat durch Zudosieren von Ammoniak bis zum Vorneutralisieren der Lösung auf ca. 1 Mol $HNO_3$/Liter und anschließendes Zusetzen von Oxalsäure und/oder Ammoniumoxalat und nachfolgendes Verringern der Acidität durch weiteres Zugeben von $NH_3$ und/oder $NH_4OH$,

Ein derartiges Verfahren ist aus der deutschen Offenlegungsschrift 26 23 353 bekannt. Es eignet sich besonders gut zum Herstellen von Americiumdioxid in gegen die Radioaktivität des Americiums abgeschirmten Räumen, indem durch das Zugeben von $NH_3$ bzw. $NH_4OH$ zur vorneutralisierten Lösung deren pH-Wert auf 2,5 eingestellt und erst die Reaktionsflüssigkeit und anschließend die entstandene Suspension noch für etwa 1 Stunde umgewälzt wird. Hierauf wird das ausgefällte Americiumoxalat abfiltriert und in einem Kalzinierofen bei 400° bis 800°C in Americiumdioxid umgewandelt, welches nach dem Abkühlen zerkleinert und gesiebt wird.

Das chemische Element Americium gehört zu den Transuranen. Es kommt in der Natur nicht vor, sondern entsteht unter anderem beim Zerfall des Plutoniumisotops Pu 241, das zusammen mit anderen Plutoniumisotopen in Wiederaufbereitungsanlagen für bestrahlte Kernreaktorbrennelemente anfällt.

Insbesondere fällt das Americium zu einem wesentlichen Teil bei der Konversion von Plutoniumnitrat zu Plutoniumdioxid an und liegt dann in der dreiwertigen Form als Americiumnitrat vor, welches mehr oder weniger metallische Verunreinigungen aufweisen kann. Als solche metallische Verunreinigungen kommen beispielsweise Aluminium, Kalzium, Chrom, Kupfer, Europium, Eisen, Gadolinium, Magnesium, Mangan, Molybdän, Natrium, Nickel und Zink sowie auch Uran und Plutonium infrage. Diese Verunreinigungen können stören, wenn das schließlich gewonnene Americiumdioxid als Strahlenquelle eingesetzt wird.

Der Erfindung liegt daher die Aufgabe zugrunde, das bekannt Verfahren weiterzubilden und die Herstellung von Americiumoxalat zu ermöglichen, das metallische Verunreinigungen, wenn überhaupt, so nur in stark vermindertem Umfang enthält.

Zur Lösung dieser Aufgabe ist ein Verfahren der eingangs erwähnten Art erfindungsgemäß dadurch gekennzeichnet, daß die Oxalsäure und/oder das Ammoniumoxalat in überstöchiometrischer Menge bezogen auf den Americiumgehalt der Americiumnitratlösung zugesetzt wird und daß anschließend die Acidität der Lösung (durch Zusetzen von $NH_3$ und/oder $NH_4OH$) auf einen pH-Wert von 2,5 bis 2,9, vorzugsweise von 2,7, herabgesetzt wird, so daß Americiumoxalat quantitativ ausfällt und Oxalatverbindungen von in der Ausgangslösung enthaltenen metallischen Verunreinigungen zumindest in einer Teilmenge in Lösung bleiben, und daß die Lösung von den Oxalatniederschlägen abdekantiert wird.

Auf diese Weise kann der größte Teil der möglichen metallischen Verunreinigungen vom ausgefällten Americiumoxalat ferngehalten werden. Günstigerweise wird die Acidität der Lösung durch das $NH_3$ und/oder $NH_4OH$ so weit herabgesetzt, daß die metallischen Verunreinugungen in größerer Menge in Lösung bleiben als ausgefällt wird.

Es ist günstig, wenn zum Abreichern der Oxalatniederschläge von Oxalaten der Metallverunreinigungen, insbesondere von Nickel-, Zink- und Kupferoxalat, diese Oxalatniederschläge mit einer wässrigen Lösung aus Ammoniumoxalat und Ammoniumhydroxid ausgewaschen werden, die einen pH-Wert im Bereich von 10 bis 10,3 hat. Diese wässrige Lösung aus Ammoniumoxalat und Ammoniumhydroxid nimmt insbesondere die Amin-Komplexbildner wie Nickel, Zink und Kupfer der möglichen metallischen Verunreinigungen auf und entfernt sie aus dem Americiumoxalatniederschlag.

Aus der US-Patentschrift 37 81 404 ist es bekannt, Americium von Kalzium- und Magnesiumverunreinigungen in salpetersaurer Lösung zu trennen, indem der pH-Wert dieser Lösung auf genau 1 eingestellt und das Americium durch Zugabe einer stöchiometrischen Menge Oxalsäure bezogen auf den Americiumgehalt in Form von Americiumoxalat ausgefällt wird. Hierbei würden aber andere Verunreinigungen wie z.B. Eisen zusammen mit dem Americium ausfallen, also nicht vom Americium getrennt werden.

Die Erfindung und ihre Vorteile seien anhand eines Ausführungsbeispieles näher erläutert:

Als Ausgangslösung wird etwa 1 Liter einer Americiumnitratlösung in 4 bis 5 molarer Salpetersäure verwendet, die die in folgender Tabelle aufgeführte Zusammensetzung hat und etwa 25 g Americium enthält:

| Element | Ausgangslösung | | Endprodukt |
|---|---|---|---|
| | Metall-verunreinig., bezogen auf Am ( % ) | Metall-verunreinig. ( g/l ) | AmO$_2$ Verunreinigungs-grad bezogen auf Am ( % ) |
| Am | 100 | 24,9 | > 97 % Am |
| Al | 3,9 | 1,0 | < 0,01 |
| Ca | 1,9 | 0,5 | < 0,01 |
| Cr | 16,0 | 4,0 | < 0,01 |
| Cu | 2,4 | 0,6 | < 0,01 |
| Eu | < 0,02 | < 0,01 | < 0,01 |
| Fe | 350 | 87 | < 0,5 |
| Gd | < 0,02 | < 0,01 | < 0,01 |
| Mg | 1,8 | 0,5 | < 0,01 |
| Mn | 3,0 | 0,75 | < 0,1 |
| Mo | 0,3 | 0,1 | < 0,01 |
| Na | 120 | 30 | < 0,01 |
| Ni | 10 | 2,5 | < 1 |
| Zn | 6 | 1,5 | < 0,1 |
| U | 6,6 | 1,6 | < 0,2 |

Diese Ausgangslösung wird in ein Reaktionsgefäß eingefüllt, das etwa 8 Liter Volumen hat. In diesem Reaktionsgefäß wird die Ausgangslösung mit Wasser verdünnt und mit NH$_3$ vorneutralisiert, bis die HNO$_3$-Konzentration etwa 1 molar ist. Hierauf wird der Lösung Oxalsäure, vorzugsweise in fester Form, in überstöchiometrischer Menge bezogen auf den Americiumgehalt zugesetzt. Günstigerweise beträgt die zugesetzte Menge Oxalsäure bis zur 20-fachen Menge des Americiumgehaltes der Americiumnitratlösung. Hierdurch bilden sich lösliche, zum Teil komplexe Oxalatverbindungen einer Reihe von möglichen metallischen Verunreinigungen, z.B. der Verunreinigungen Natrium, Eisen, Chrom und Nickel.

**0 121 702**

Nach dem Auflösen der Oxalsäure wird die Acidität der Lösung durch Einleiten von $NH_3$ auf einen pH-Wert von 2,3 bis 2,9, vorzugsweise von 2,7 herabgesetzt. Die Temperatur der Lösung wird auf etwa 60°C gehalten.

Nachdem sich dieser pH-Wert eingestellt und der Americiumoxalatniederschlag abgesetzt hat, wird die überstehende Lösung, die eine Reihe metallischer Verunreinigungen der Ausgangslösung in Form von Oxalatverbindungen gelöst enthält, abdekantiert. Anschließend wird der Americiumoxalatniederschlag zweimal mit 0,1 molarer Oxalsäurelösung gewaschen und die Waschlösung abdekantiert.

Günstigerweise wird der Americiumoxalatniederschlag, der noch ca. 10 bis 20 % Restverunreinigungen enthalten kann, noch einmal in ca. 3 Liter 13 bis 13 molarer Salpetersäure aufgenommen, die ca. 60°C warm ist und auf den Niederschlag etwa 2 bis 3 Stunden einwirken kann. Sodann wird die salpetersaure Americiumnitratlösung.auf 6,3 Liter mit Wasser verdünnt und erneut $NH_3$ eingeleitet. Sobald die Lösung einen pH-Wert von 0,3 erreicht hat, wird $(NH_4)_2C_2O_4$ der Lösung im Verhältnis der 1,3-fachen Gewichtsmenge bezogen auf das in der Lösung enthaltene Americium zugesetzt. Anschließend wird weiter $NH_3$ zudosiert und der pH-Wert der Lösung auf 1,0 eingestellt. Die Temperatur der Lösung wird hierbei auf etwa 60°C gehalten.

Es bildet sich wieder ein Americiumoxalatniederschlag, nach dessen Absetzen die überstehenden, die metallischen Verunreinigungen aus der Ausgangslösung gelöst enthaltende Lösung abdekantiert wird. Der Americiumoxalatniederschlag wird sodann ein oder zweimal mit 0,1 molarer Oxalsäure gewaschen.

Zur Abreicherung der Amin-Komplexbildner, wie z.B. Zink, Nickel und Kupfer, unter den möglichen metallischen Verunreinigungen aus der Ausgangslösung wird der Americiumoxalatniederschlag sodann mit einer 0,2 molaren wässrigen Ammoniumoxalatlösung versetzt. Unter Rühren der Reaktionsflüssigkeit wird sodann durch Einleiten von $NH_3$ der pH-Wert auf 10 bis 10,3 eingestellt und die Reaktionsflüssigkeit anschließend noch 3 Stunden lang gerührt. Nach dem Absetzen des Americumoxalatniederschlages wird sodann die überstehende klare Lösung abdekantiert.

Mit dieser überstehenden klaren Lösung werden die Metalle Zink, Nickel und Kupfer als Ammonium-Metalloxalate, z.B. $(NH_4)_i$ $Me(C_2O_4)_j$ (Me = Zn, Ni oder Cu; i = 2 oder 4; j = 2 oder 3) und als komplexe Aminverbindungen, z.B. Zn $[(NH_3)_4]$ $C_2O_4$ oder Ni $[NH_3]_x$ $C_2O_4$ mit x = 2 bis 6 aus dem Americumoxalatniederschlag durch Auswaschen entfernt.

Dieser Auswaschvorgang kann noch 1 bis 2 mal wiederholt werden. Sodann wird der Americiumoxalatniederschlag über einen Quarzglasfilter abfiltriert und mehrmals mit 0,2 molarer Ammoniumoxalatlösung, die einen pH-Wert von 10 hat, gewaschen.

Ein aus diesem Americumoxalatniederschlag durch Kalzinieren bei 400° bis 800°C als Endprodukt gewonnenes Americiumdioxid hat die in der oben angeführten Tabelle angegebenen Verunreinigungsgrade.

Wie man erkennt, hat das gewonnene Americiumdioxid einen Reinheitsgrad größer als 97 % Americium. Dieser Reinheitsgrad wurde durch ein unkompliziertes Ausfäll- und Reinigungsverfahren für Americiumoxalat erzielt, das nur einen geringen apparativen Aufwand erfordert, bei dem nur ein relativ geringes Volumen an wässrigem radioaktiven Abfall anfällt und das in abgeschirmten Räumen, z.B. in heißen Zellen, ohne Schwierigkeiten durchgeführt werden kann. Insbesondere entfällt jeglicher organischer radioaktiver Abfall.

**Patentansprüche**

1. Verfahren zum Ausfällen von Americiumoxalat aus einer salpetersauren Ausgangslösung von Americiumnitrat durch Zudosieren von Ammoniak bis zum Vorneutralisieren der Lösung auf ca. 1 Mol $HNO_3$/Liter und anschließendes Zusetzen von Oxalsäure und/oder Ammoniumoxalat und nachfolgendes Verringern der Acidität durch weiteres Zugeben von $NH_3$ und/oder $NH_4OH$, dadurch gekennzeichnet, daß die Oxalsäure und/oder das Ammoniumoxalat in überstöchiometrischer Menge bezogen auf den Americiumgehalt der Americiumnitratlösung zugesetzt wird und daß anschließend die Acidität der Lösung durch Zusetzen von $NH_3$ und/oder $NH_4OH$ auf enen pH-Wert von 2,5 bis 2,9, vorzugsweise von 2,7, herabgesetzt wird, daß Americiumoxalat quantitativ ausfällt und Oxalatverbindungen von in der Ausgangslösung enthaltenen metallischen Verunreinigungen zumindest in einer Teilmenge in Lösung bleiben, und daß die Lösung von den Oxalatniederschlägen abdekantiert wird.

2. Verfahren nach Anspruch 1, dadurch gekennzeichnet, daß zum Abreichern der Oxalatniederschläge von Oxalaten der Metallverunreinigungen, insbesondere von Nickel-, Zink- und Kupferoxalat, diese Oxalatniederschläge mit einer wässrigen Lösung aus Ammoniumoxalat und Ammoniumhydroxid ausgewaschen werden,die einen pH-Wert im Bereich von 10 bis 10,3 hat.

**Claims**

1. A process for the precipitation of americium oxalate from a starting nitric acid solution, of americium nitrate by adding ammonia up to the preliminary neutralization of the solution at about 1 mol $HNO_3$/litre and subsequently adding oxalic acid and/or ammonium oxalate, and by subsequently reducing the acidity by the further addition of $NH_3$ and/or $NH_4OH$, characterised in that the oxalic acid and/or the ammonium oxalate are added in an super-stoichiometrical amount relative to the

4

americium content of the americium nitrate solution; that the acidity of the solution is subsequently reduced by adding $NH_3$ and/or $NH_4OH$ to a pH-value of 2.5 to 2.9, preferably 2.7, so that americium oxalate is quantitatively precipitated and at least a partial amount of oxalate compounds of metallic impurities, which are contained in the starting solution, remains in the solution; and that the solution is decanted from the oxalate precipitates.

2. A process as claimed in Claim 1, <u>characterised in</u> that, in order to deplete the oxalate precipitates of oxalates of the metallic impurities, in particular of nickel oxalate, zinc oxalate and copper oxalate, said oxalate precipitates are washed with an aqueous solution of ammonium oxalate and ammonium hydroxide which has a pH-value in the range of 10 to 10.5.

**Revendications**

1. Procédé de précipitation d'oxalate d'américium dans une solution de départ nitrique de nitrate d'américium, par addition dosée d'ammoniac jusqu'à la neutralisation préalable de la solution à environ 1 mole de $HNO_3$/litre, puis par addition d'acide oxalique et/ou d'oxalate d'ammonium et par diminution ultérieure de l'acidité en ajoutant encore du $NH_3$ et/ou du $NH_4OH$, caractérisé en ce qu'on ajoute l'acide oxalique et/ou l'oxalate d'ammonium en une quantité supérieure à la quantité stoechiométrique, rapportée à la teneur en américium de la solution de nitrate d'américium, puis on diminue l'acidité de la solution par addition de $NH_3$ et/ou de $NH_4OH$ jusqu'à obtention d'un pH de 2,5 à 2,9 et, de préférence, de 2,7, de sorte que l'oxalate d'américium précipite quantitativement et que des composés d'oxalates d'impuretés métalliques contenues dans la solution de départ restent au moins en quantité partielle dans la solution et on sépare les précipités d'oxalates de la solution par décantation.

2. Procédé suivant la revendication 1, caractérisé en ce que, pour appauvrir les précipités d'oxalates en oxalates d'impuretés métalliques, en particulier en oxalate de nickel, en oxalate de zinc et en oxalate de cuivre, on sépare ces précipités d'oxalates par lavage en une solution aqueuse d'oxalate d'ammonium et d'hydroxyde d'ammonium ayant un pH allant de 10 à 10,5.